# EUROPEAN PATENT APPLICATION

(11) **EP 2 433 580 A1**
(43) Date of publication of application: **28.03.2012**
(21) Application number: 10011275.4
(22) Date of filing: 28.09.2010
(51) Int. Cl.: A61B 17/70, A61B 17/80

(54) **An anterior spinal stabilization system**

(71) Applicant: Zimmer GmbH, 8404 Winterthur (CH)
(72) Inventor: Vonwiller, Stephan, 8404 Winterthur (CH); Fröhlich, Markus, 8362 Balterswil (CH)
(74) Representative: Manitz, Finsterwald & Partner GbR

(57) **Abstract**

The present application relates to an anterior spinal stabilization system comprising: at least one vertebral plate (11) including a cranial end (19), a caudal end (21), a longitudinal axis (23) defined by said cranial and caudal ends (19, 21), an anterior surface (41), a posterior surface (43), a longitudinal section along said longitudinal axis (23) defined by said anterior and posterior surfaces (41, 43), a first section (13) terminating in said cranial end (19) and being configured to be attached to an anterior face (39) of a vertebral body of a first vertebra (33), a second section (15) terminating in said caudal end (21) and being configured to be attached to an anterior face (39) of a vertebral body of a second vertebra (35), and a middle section (17) positioned between said first and second sections (13, 15), wherein said longitudinal section is multiple-curved along said longitudinal axis (23).

## Description

The present disclosure relates to an anterior spinal stabilization system comprising at least one vertebral plate.

For treating damage to a spine due to degeneration, aging, disease or physical trauma various types of static and dynamic vertebral plates implanted during surgery are currently used to connect and thus stabilize the affected vertebrae. A typical static vertebral plate is a rigid single piece of metal allowing no movement between the plate-mounted vertebrae, thereby limiting mobility. This, however, is not always desired. A typical dynamic vertebral plate includes two plate pieces which are slidably engaged to each other to form the vertebral plate and to allow movement between the plate-mounted vertebrae. This may be desirable in cases where the height of the intervertebral space between the plate-mounted vertebrae changes, e.g. due to subsidence of an interbody spacer replacing a removed disk. Such a dynamic vertebral plate, however, does not allow adequate spinal flexion and extension.

Accordingly, there is a need for an improved anterior spinal stabilization system.

The present disclosure provides an anterior spinal stabilization system comprising at least one vertebral plate. The vertebral plate includes a cranial end, a caudal end, and a longitudinal axis defined by the cranial and caudal ends. The vertebral plate further includes an anterior surface, a posterior surface and a longitudinal section along the longitudinal axis defined by the anterior and posterior surfaces. The vertebral plate further includes a first section terminating in the cranial end and being configured to be attached to an anterior face of a vertebral body of a first vertebra, and a second section terminating in the caudal end and being configured to be attached to an anterior face of a vertebral body of a second vertebra. The vertebral plate further includes a middle section positioned between the first and second sections. The longitudinal section is multiple-curved along the longitudinal axis.

The multiple-curved shape of the vertebral plate stabilizes the plate-mounted vertebrae and, at the same time, allows motion therebetween. The multiple-curved shape of the vertebral plate allows flexion and extension even in the segment of the spine which comprises the plate-mounted vertebrae. Due to its multiple-curved shape the vertebral plate is designed as a flexible vertebral plate. A loading upon the vertebral plate in cranial-caudal direction may be redirected into anterior-posterior direction owing to the curved shape of the vertebral plate.

In an aspect, each of the first and second sections has a curvature, the direction of curvature of the first section corresponding to the direction of curvature of the second section, and the middle section or at least a portion thereof being counter-curved thereto. The middle section may have more than one curve, in particular three curves, following each other along the longitudinal direction. The direction of curvature may turn from one curve to the next curve. When the middle section includes three curves, a middle curve thereof positioned between cranial and caudal curves of the middle section may be curved in the same direction as the first and second sections. A curve's curvature does not have to be constant but may be at least substantially constant in particular cases.

In another aspect, each of the first and second sections is concave at the anterior surface and is convex at the posterior surface, and wherein the middle section or at least a portion thereof is convex at the anterior surface and is concave at the posterior surface.

In still another aspect, the vertebral plate has a thickness defined by the anterior and posterior surfaces, the thickness tapering from the middle section towards the first and second sections and/or towards the cranial and caudal ends. Thus, the flexibility of the plate may increase from the middle section towards the first and second sections and/or towards the cranial and caudal ends.

In yet another aspect, the middle section is configured to allow axial movement of the first and second sections along the longitudinal axis with respect to each other. This may be attained by the middle section including a curve having a high curvature. Allowing axial movement of the first and second sections with respect to each other may be beneficial owing to the fact that the vertebral plate is positioned anterior to the natural center of rotation of the spine.

In a further aspect, the anterior spinal stabilization system further comprises at least one top plate mountable or mounted to the vertebral plate, preferably to the anterior surface of the vertebral plate. The vertebral plate may then be denoted as a base plate.

In an aspect, the system has a higher bending stiffness against loading in anterior-posterior direction than the vertebral plate. The top plate may be a flexurally rigid plate which completely suppresses the flexibility of the vertebral plate when mounted thereto and thus suppresses the flexion and extension ability of the spine in the segment of the spine which comprises the plate-mounted vertebrae for fusing the plate-mounted vertebrae.

In another aspect, the top plate is smaller in size than the vertebral plate, in particular has a smaller length along the longitudinal axis than the vertebral plate. A longitudinal section of the top plate may be single-curved along a longitudinal axis of the top plate.

In still another aspect, the top plate has a posterior surface, each of the anterior surface of the middle section of the vertebral plate and the posterior surface of the top plate having a curvature, the curvatures being substantially identical to each other in an unloaded state of the vertebral and top plates.

In yet another aspect, the top plate is mountable to and removable from the vertebral plate while the vertebral plate is fixed to the vertebral bodies of the first and second vertebras. Thus, if the health condition of the degenerated, aged, diseased or traumatized plate-mounted spinal segment worsens with time requiring higher stabilizing support from the anterior spinal stabilization system, during revision surgery a top plate may be added to the vertebral plate which was implanted during first-time surgery or an existing top plate may be replaced by another top plate having a higher stiffness against loading in anterior-posterior direction. If, however, the damaged plate-mounted spinal segment recovers, during revision surgery an existing top plate may be removed or, as the case may be, replaced by another top plate having a lower stiffness.

In a further aspect, the anterior spinal stabilization system further comprises a plurality of top plates, each of which being mountable to the vertebral plate, the top plates having bending stiffnesses differing from each other. Thus, the top plate to be implanted may be chosen in accordance with the patient's anatomy and health condition (e.g. degree of degeneration) of the plate-mounted spinal segment.

In an aspect, the vertebral plate, in particular each of the first and second sections of the vertebral plate may be configured to allow multi-level spinal stabilization.

In another aspect, each of the first and second sections of the vertebral plate is configured to allow stacking of the first and second sections with respective second and first sections of a second such vertebral plate in an overlapping manner.

In still another aspect, each of the first and second sections of the vertebral plate comprises at least one segment having a through hole for receiving a bone screw, the segment of the first section being provided with a recess on one of the anterior and posterior surfaces and the segment of the second section being provided with a recess on the one of the anterior and posterior surfaces or on the other one of the anterior and posterior surfaces to allow mating engagement of the vertebral plate with the second such vertebral plate.

In yet another aspect, each of the first and second sections of the vertebral plate comprises at least one through hole.

In a further aspect, the anterior spinal stabilization system further comprises a second such vertebral plate and/or a bone screw for each of the through holes, and, in particular, a cap screw for each of the bone screws, each of the cap screws being configured to engage the bone screw.

It will be appreciated that the specific features of the embodiments described above can be combined. Thus any combinations of the features described in the dependent claims are disclosed herein, be they explicitly mentioned or not.

The above-mentioned and other features and advantages of this disclosure, and the manner of attaining them, will become more apparent and the disclosure itself will be better understood by reference to the following description of several embodiments of the disclosure taken in conjunction with the accompanying drawings, wherein:
Fig. 1 is a perspective view of an anterior vertebral plate of the present disclosure according to one exemplary embodiment;
Fig. 2 is a side view of the anterior vertebral plate of Fig. 1;
Fig. 3 is a side view of the anterior vertebral plate of Fig. 1 depicting the anterior vertebral plate in an unloaded state and loaded (flexion and extension) states;
Fig. 4 is a side view of an anterior vertebral plate of the present disclosure according to another exemplary embodiment;
Fig. 5 is a side view of an anterior vertebral plate of the present disclosure according to another exemplary embodiment;
Fig. 6 is a top view of an anterior spinal stabilization system of the present disclosure according to one exemplary embodiment comprising an anterior vertebral plate and a top plate;
Fig. 7 is a side view of the anterior spinal stabilization system of Fig. 6;
Fig. 8 is a top view of the anterior vertebral plate of Fig. 6 adjoining to second and third such anterior vertebral plates;
Fig. 9 is a top view of an anterior spinal stabilization system of the present disclosure according to another exemplary embodiment comprising two anterior vertebral plates;
Fig. 10 is a fragmentary side view of the anterior spinal stabilization system of Fig. 9;
Fig. 11 is a top view of an anterior vertebral plate of the present disclosure according to another exemplary embodiment; and
Fig. 12 is a cross-sectional view along A-A in Fig. 11.

The following description is merely exemplary in nature and is not intended to limit the present disclosure, application, or uses. It should be understood that throughout the drawings, corresponding reference numerals indicate like or corresponding parts and features.

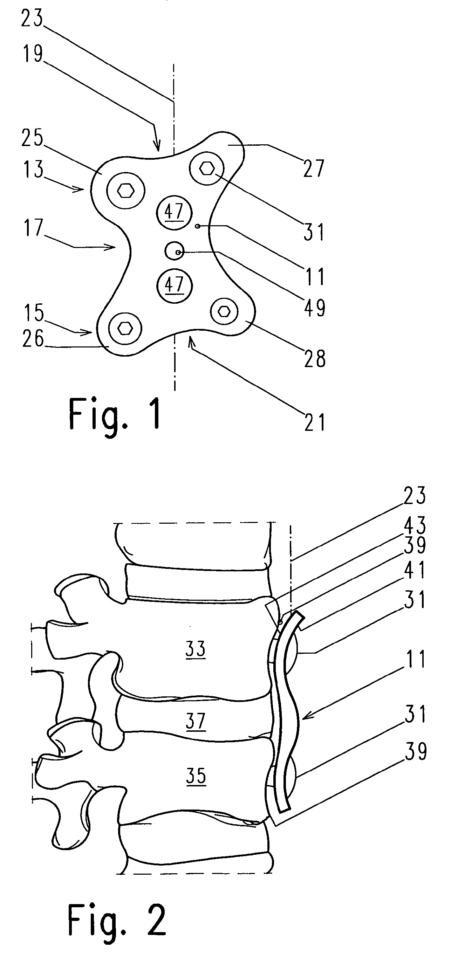

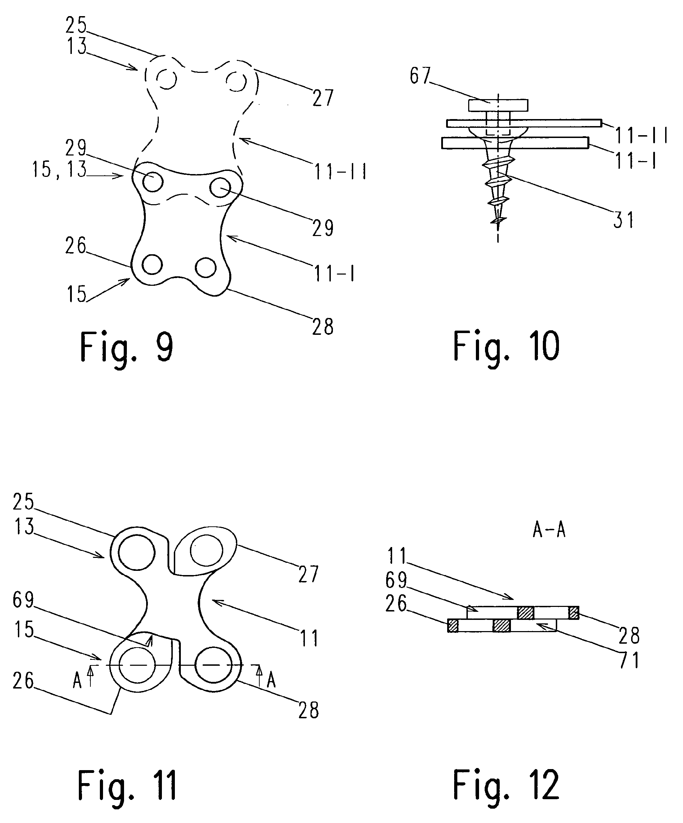

Fig. 1 shows a leaf-spring like anterior vertebral plate 11 made from e.g. carbon or polyetheretherketone (PEEK) which comprises a first section 13 terminating in a cranial end 19 and a second section 15 terminating in a caudal end 21 of the plate 11. The cranial and caudal ends 19, 21 define a longitudinal axis 23. A middle section 17 of the anterior vertebral plate 11 is positioned between the first and second sections 13, 15. In the top view or front view of Fig. 1, the plate 11 is roughly X-shaped. The first section 13 includes two segments 25, 27 each of which is provided with a bore (cf. Fig. 9) for receiving a headed bone screw 31. The second section 15 includes two segments 26, 28 each of which is provided with a bore for receiving a headed bone screw 31.

Fig. 2 is a fragmentary side view of a spinal column including first and second vertebrae 33, 35 and a spinal disk 37 positioned therebetween. Each of the first and second vertebrae 33, 35 include a vertebral body having an anterior face 39. The plate 11 having anterior and posterior surfaces 41, 43 is configured to be attached to the anterior faces 39 of the first and second vertebrae 33, 35 to support the spinal column in that region. The plate 11 is fixed to the spinal column via the headed bone screws 31.

The anterior and posterior surfaces 41, 43 define a longitudinal section of the plate 11 that extends along the longitudinal axis 23. The longitudinal section is multiple-curved along the longitudinal axis 23 just as a recurve bow. The plate 11 is made of a flexible material having an elasticity that allows bending of the longitudinal section upon loading in cranial-caudal-direction or anterior-posterior direction. Thus, the plate 11 allows flexion and extension at the joint defined between the first and second vertebrae 33, 35 upon placing strain upon the plate 11.

The multiple-curved shape of the plate 11 in lateral viewing direction is realized by each of the first, second and middle sections 13, 15, 17 having a curvature. The curvatures of the first and second sections 13, 15 are curved in the same direction, namely concave at the anterior surface 41 and convex at the posterior surface 43. The direction of the curvature of the middle section 17 is opposite to the direction of the curvatures of the first and second sections 13, 15, i.e. convex at the anterior surface 41 and concave at the posterior surface 43. The first and middle sections 13, 17 and the middle and second sections 17, 15, respectively, pass over into each other steadily and/or smoothly, i.e. without any discontinuity or edge.

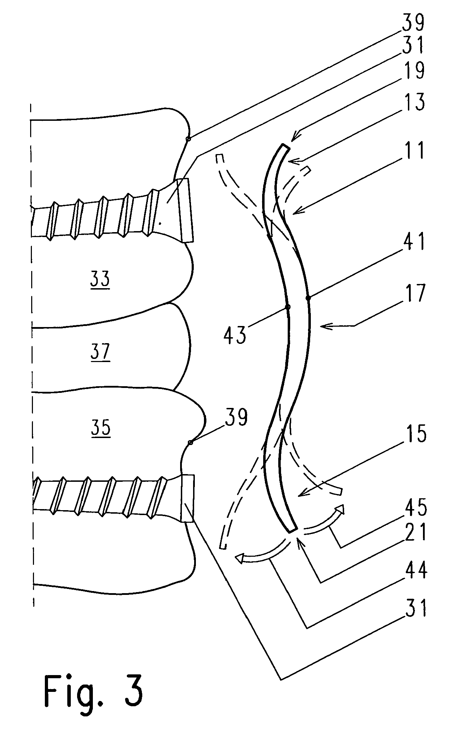

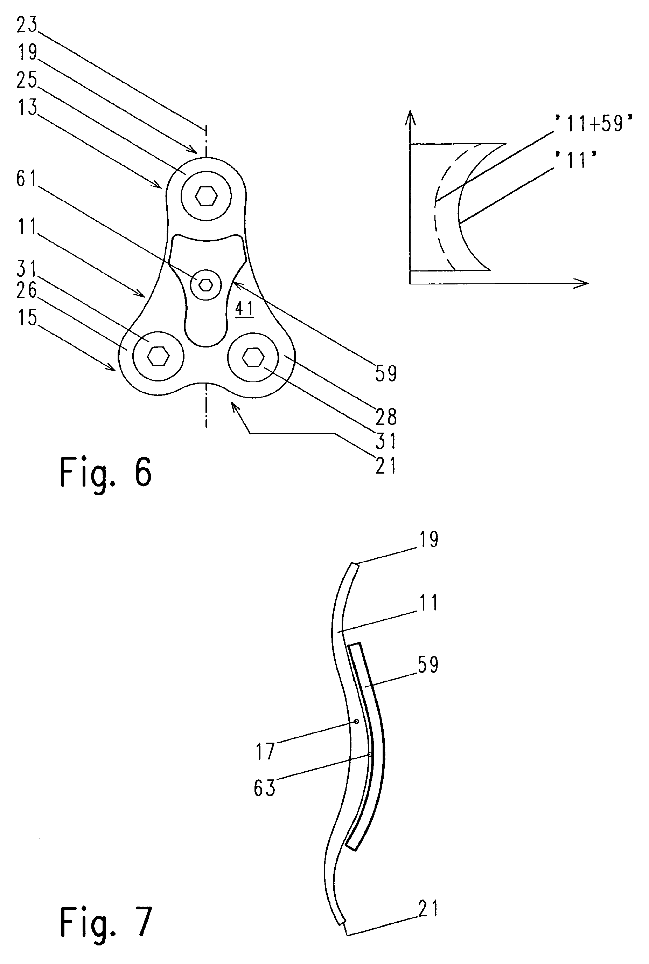

As shown in Fig. 3, the plate 11 (which is spaced apart from the spinal column for illustration purposes only) may be configured such that bending of the plate 11, namely extension 44 and flexion 45, occurs mainly in the transitional regions between the middle and first sections 17, 13 and the middle and second sections 17, 15. This may be achieved by the thickness of the plate 11 tapering from the middle section 17 towards said cranial and caudal ends 19, 21 which thickness is defined by the anterior and posterior surfaces 41, 43 of the plate 11. Each of the transitional regions may optionally be provided with at least one bore 47 (cf. Fig. 1) to affect or adjust the local flexibility of the plate 11. Further, the middle section 17 may be provided with a bore 49 (cf. Fig. 1) for mounting a top plate to the plate 11 as will be described in more detail in connection with Figs. 6-8 below.

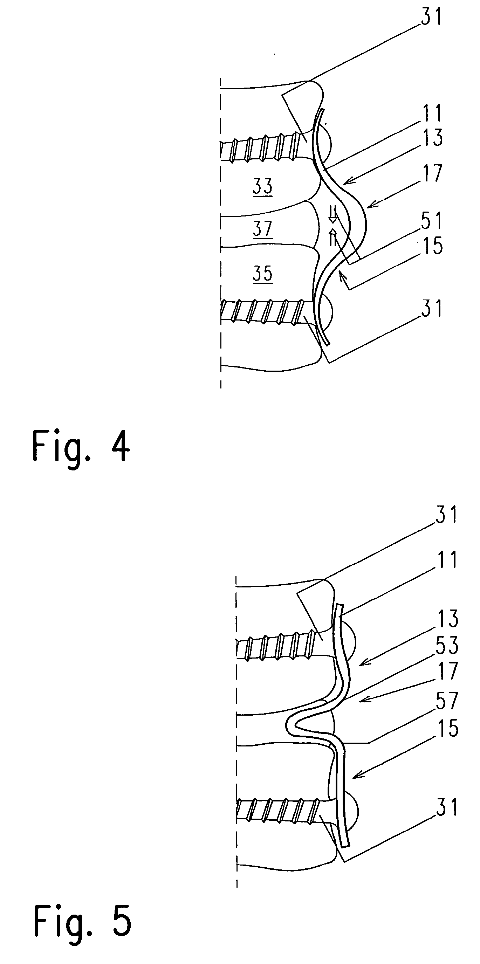

In the embodiment of Fig. 4, the middle section 17 of the plate 11 has a higher curvature than the middle section 17 of the plate 11 according to the embodiment of Fig. 2. Thus, the plate 11 of the embodiment according to Fig. 4 allows a higher amount of axial movement (illustrated by the arrows 51) of the first and second vertebrae 33, 35 with respect to each other than plate 11 of Fig. 2. The first and section sections 13, 15 of the plate 11 of Fig. 4 are configured at least substantially identical to the first and second sections 13, 15 of the plate 11 of Figs. 2.

The plate 11 of the embodiment according to Fig. 5 comprises a multiple-curved middle section 17 having cranial, middle and caudal portions 53, 55, 57 adjoining each other, each of which being configured as a curve. In total, the plate 11 of Fig. 5 has a higher number of curves than the plates 11 according to the embodiments of Figs. 2 and 4. The direction of curvature of the middle portion 55 is the same as the direction of curvature of the first and second sections 13, 15. The cranial and caudal portions 53, 57 are counter-curved to the middle portion 55. The first and section sections 13, 15 of the plate 11 of Fig. 5 are configured at least substantially identical to the first and second sections 13, 15 of the plates 11 of Figs. 2 and 4.

The anterior spinal stabilization system according to Fig. 6 comprises an anterior vertebral plate 11 configured as a base plate and a top plate 59 made from e.g. carbon reinforced or pure polyetheretherketone (PEEK) mounted to the anterior surface 41 of the plate 11 via a set screw 61 extending through a bore provided in the top plate 59 and engaging a bore provided in the plate 11. The plate 11 is roughly triangle-shaped. Unlike the first section 13 of the plate 11 according to Fig. 2, the first section 13 of the plate 11 of Fig. 6 comprises only one segment 25 which is provided with a bore for receiving a headed bone screw 31. The second section 15, however, includes two such segments 26, 28 as does the plate 11 according to Fig. 2. The size of the top plate 59 is smaller than the size of the plate 11 and the top plate 59 has a smaller length along the longitudinal axis 23 than the plate 11. In particular, the top plate 59 does not project over the plate 11 and the length of the top plate 11 approximately corresponds to the length of the middle section 17 of the plate 11.

Fig. 6 includes a schematic diagram showing the flexibility x of the plate 11 and of the anterior spinal stabilization system comprising the plate 11 and the top plate 59 in anterior-posterior direction in dependence upon position y. According to the diagram, the flexibility of the plate 11 alone is higher than the flexibility of the system additionally including the top plate 59. In other words, the bending stiffness of the plate 11 against loading in anterior-posterior direction is lower than the bending stiffness of the system including the plate 11 and the top plate 59. The shape of the graph of the diagram of Fig. 6 depends upon the cross-section of the plate 11 and/or of the top plate 59.

In both cases, the flexibility increases towards the cranial and caudal ends 19, 21 of the plate 11 due to the tapering of the plate 11 towards these ends 19, 21 which tapering can be taken from Fig. 7. The top plate 59 (which is spaced apart from the plate 11 for illustration purposes only) is single-curved and includes a posterior surface 63. At least in an unloaded or unstrained state of the anterior spinal stabilization system and optionally also in a loaded or strained state, the curvature of the top plate 59 corresponds to the curvature of the middle section 17 of the plate 11. Thus, the top plate 59 may be snugly attached to the plate 11.

The anterior spinal stabilization system according to Fig. 6 includes a plurality of top plates (not shown). Each of the top plates is mountable to the plate 11 as described above. The top plates have flexibilities and/or bending stiffnesses differing from each other. Thus, it is possible to choose a top plate which leads to an overall flexibility and/or bending stiffness which suits best to the needs of the plate-mounted spinal segment.

The (respective) top plate 59 may be mounted to and removed from the plate 11 while the plate 11 is fixedly attached to the vertebral bodies of the first and second vertebrae 33, 35. This allows replacing an implanted top plate having a first flexibility by another top plate having a second flexibility different from the first flexibility during revision surgery. Such replacement may be desired when the health condition of the plate-mounted spinal segment 33, 35 changes (e.g. gets better or worse).

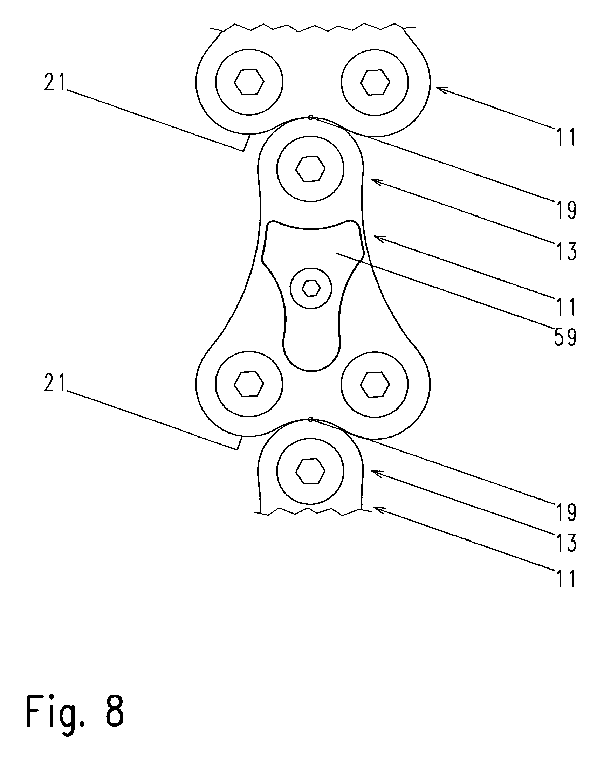

As indicated in Fig. 8, the plate 11 of the embodiment according to Fig. 6 and other such plates 11 may be used for multi-level spinal stabilization. Together, the plates 11 span and thus stabilize more than two consecutive vertebrae. The consecutive plates 11 abut each other but do not overlap each other. In particular, rounded cranial end 19 of the first section 13 of the plate 11 engages counter-shaped caudal end 21 of another such plate 11. In Fig. 8, the middle plate 11 is provided with a top plate 59.

In the embodiment according to Figs. 9 and 10, the plate 11-I is configured to allow stacking of the first and second sections 13, 15 with respective second and first sections 15, 13 of a second such plate 11-II by overlapping the plates 11. In particular, the segments 25, 27 of the first section 13 of the plate 11-I overlap with the segments 26, 28 of the second section 15 of the plate 11-II such that the bores 29 provided in the segments 25 and 26 and 27 and 28, respectively, lie on top of each other. In the implanted state, the first section 13 of plate 11-I is anchored to the respective vertebra via the bores 29 provided in the first section 13 and via the headed bone screws 31. A head 65 of the respective bone screw 31 is positioned between the plate 11-I and the plate 11-II. The second section 15 of the plate 11-II is secured to the first section 13 of the plate 11-I via the bores 29 provided in the second section 15 and via cap screws 67 which engage the heads 65 of the respective bone screws 31.

In the embodiment according to Figs. 11 and 12, each of the first and second sections 13, 15 of the plate 11 of the anterior spinal stabilization system comprises two segments 25, 27 and 26, 28, respectively, a first 27, 26 of which being provided with a recess 69 on one of the anterior and posterior surfaces 41, 43 and a second 25, 28 of which being provided with a recess 71 on the other one of the anterior and posterior surfaces 43, 31, the recesses 69 of the first segments 27, 26 of the first and second sections 13, 15 being provided on a same of the anterior and posterior surfaces 41, 43, the first segment 27 of the first section 13 and the second segment 28 of the second section 15 being positioned on a same lateral side of the plate 11 and the second segment 25 of the first section 13 and the first segment 26 of the second section 15 being positioned on the other lateral side of the plate 11. The plate 11 allows mating engagement with a second such plate 11 for multi-level spinal stabilization.

While this disclosure has been described illustrated by the description of one or more embodiments thereof, they are not intended to restrict or in any way limit the scope of this disclosure. This application is therefore intended to cover any variations, uses, or adaptations of the invention using its general principles. Further, this application is intended to cover such departures from the present disclosure as come within known or customary practice in the art to which this disclosure pertains and which fall within the limits of the appended claims.

### List of Reference Numerals:

- 11: anterior vertebral plate
- 13: first section
- 15: second section
- 17: middle section
- 19: cranial end
- 21: caudal end
- 23: longitudinal axis
- 25: segment of first section
- 26: segment of second section
- 27: segment of first section
- 28: segment of second section
- 29: bore
- 31: bone screw
- 33: first vertebra
- 35: second vertebra
- 37: spinal disk
- 39: anterior face
- 41: anterior surface
- 43: posterior surface
- 44: extension
- 45: flexion
- 47: bore
- 49: bore
- 51: arrow
- 53: cranial portion
- 55: middle portion
- 57: caudal portion
- 59: top plate
- 61: set screw
- 63: posterior surface
- 65: head
- 67: cap screw
- 69: recess
- 71: recess

## Claims

1. An anterior spinal stabilization system comprising:
at least one vertebral plate (11) including a cranial end (19), a caudal end (21), a longitudinal axis (23) defined by said cranial and caudal ends (19, 21), an anterior surface (41), a posterior surface (43), a longitudinal section along said longitudinal axis (23) defined by said anterior and posterior surfaces (41, 43), a first section (13) terminating in said cranial end (19) and being configured to be attached to an anterior face (39) of a vertebral body of a first vertebra (33), a second section (15) terminating in said caudal end (21) and being configured to be attached to an anterior face (39) of a vertebral body of a second vertebra (35), and a middle section (17) positioned between said first and second sections (13, 15),
wherein said longitudinal section is multiple-curved along said longitudinal axis (23).

2. The anterior spinal stabilization system of claim 1, wherein each of said first and second sections (13, 15) has a curvature, the direction of curvature of the first section (13) corresponding to the direction of curvature of the second section (15), and said middle section (17) or at least a portion (53, 57) thereof being counter-curved thereto.

3. The anterior spinal stabilization system of claim 1 or claim 2, wherein each of said first and second sections (13, 15) is concave at said anterior surface (41) and is convex at said posterior surface (43), and wherein said middle section (17) or at least a portion thereof (53, 57) is convex at said anterior surface (41) and is concave at said posterior surface (43).

4. The anterior spinal stabilization system of any one of claims 1 to 3, wherein said vertebral plate (11) has a thickness defined by said anterior and posterior surfaces (41, 43), said thickness tapering from said middle section (17) towards said first and second sections (13, 15) and/or towards said cranial and caudal ends (19, 21).

5. The anterior spinal stabilization system of any one of claims 1 to 4, wherein said middle section (17) is configured to allow axial movement (51) of said first and second sections (13, 15) along the longitudinal axis with respect to each other.

6. The anterior spinal stabilization system of any one of claims 1 to 5, further comprising at least one top plate (59) mountable or mounted to said vertebral plate (11), preferably to said anterior surface (41) of said vertebral plate (11).

7. The anterior spinal stabilization system of claim 6, wherein said system (11, 59) has a higher bending stiffness against loading in anterior-posterior direction than said vertebral plate (11).

8. The anterior spinal stabilization system of claim 6 or claim 7, wherein said top plate (59) is smaller in size than said vertebral plate (11), in particular has a smaller length along said longitudinal axis (23) than said vertebral plate (11).

9. The anterior spinal stabilization system of any one of claims 6 to 8, wherein said top plate (59) has a posterior surface (63), each of said anterior surface (41) of said middle section (17) of said vertebral plate (11) and said posterior surface (63) of said top plate (59) having a curvature, said curvatures being substantially identical to each other in an unloaded state of the vertebral and top plates (11, 59).

10. The anterior spinal stabilization system of any one of claims 6 to 9, wherein said top plate (59) is mountable to and removable from said vertebral plate (11) while said vertebral plate (11) is fixed to said vertebral bodies of said first and second vertebras (33, 35).

11. The anterior spinal stabilization system of any one of claims 6 to 10, further comprising a plurality of top plates, each of which being mountable to said vertebral plate (11), said top plates having bending stiffnesses differing from each other.

12. The anterior spinal stabilization system of any one of claims 1 to 11, wherein each of said first and second sections (13, 15) of said vertebral plate (11) is configured to allow stacking of said first and second sections (13, 15) with respective second and first sections (15, 13) of a second such vertebral plate (11) in an overlapping manner.

13. The anterior spinal stabilization system of claim 12, wherein each of said first and second sections (13, 15) of said vertebral plate (11) comprises at least one segment (25, 27; 26, 28) having a through hole (29) for receiving a bone screw (31), said segment (25, 27) of said first section (13) being provided with a recess (69) on one of said anterior and posterior surfaces (41, 43) and said segment (26, 28) of said second section (15) being provided with a recess (71) on the one of said anterior and posterior surfaces (41, 43) or on the other one of said anterior and posterior surfaces (41, 43) to allow mating engagement of said vertebral plate (11) with said second such vertebral plate (11).

14. The anterior spinal stabilization system of any one of claims 1 to 13, wherein each of said first and second sections (13, 15) of said vertebral plate (11) comprises at least one through hole (29).

15. The anterior spinal stabilization system of claim 14, further comprising a second such vertebral plate (11) and/or a bone screw (31) for each of said through holes (29), and, in particular, a cap screw (67) for each of said bone screws (31), each of said cap screws (67) being configured to engage said bone screw (31).
